# EUROPEAN PATENT APPLICATION

(11) **EP 1 767 935 A1**
(43) Date of publication of application: **28.03.2007**
(21) Application number: 05743752.7
(22) Date of filing: 25.05.2005
(51) Int. Cl.: G01N 33/48, A61B 5/15

(54) **BIOLOGICAL SAMPLE COLLECTING IMPLEMENT AND METHOD OF COLLECTING BIOLOGICAL SAMPLE**

(30) Priority: 27.05.2004 JP 2004158290
(71) Applicant: EIKEN KAGAKU KABUSHIKI KAISHA, Bunkyo-ku, Tokyo 113-8408 (JP)
(72) Inventor: SEKINE, Kazuhito, Eiken Kagaku K. K., Tokyo 1300026 (JP); HIRAHARA, Shin, Eiken Kagaku K. K., Tokyo 1300026 (JP); ICHIKAWA, Yoshiharu, Eiken Kagaku K. K., Tokyo 1300026 (JP); TAKAHASHI, Tomoyoshi, Eiken Kagaku K. K., Tokyo 1300026 (JP); OYAMA, Takao, Eiken Kagaku K. K. s, Tokyo 1300026 (JP); TAKAHASHI, Masami, Eiken Kagaku K. K., Tokyo 1300026 (JP)
(74) Representative: Gleiss & Grosse
(86) International application number: PCT/JP2005/009534
(87) International publication number: WO 2005/116641

(57) **Abstract**

This invention provides a tool for recovering biological samples for realizing more simple and accurate clinical testing with the use of a trace amount of the blood sample, a kit comprising the same, and a method for recovering and separating biological samples using the same. Such tool comprises: a recovery container body composed of an upper container portion having a medium-accommodating portion, provided with an upper opening, for accommodating and conserving an absorption medium to be impregnated with the recovered biological samples and a lower container portion for accommodating the biological samples separated from the medium; and a lid for closing the upper opening of the medium-accommodating portion, the bottom of such medium-accommodating portion being provided with an open area in communication with the lower container portion.

## Description

### Technical Field

The present invention relates to a tool for efficiently and easily recovering and separating biological samples that are required for clinical testing. The present invention also relates to a method for recovering biological samples using such tool.

### Background Art

In the past, clinical chemical testing required blood sampling and sample separation, which were to be conducted by specialists, such as doctors, nurses, or clinical technologists. This required the test subjects to visit medical institutions for testing. Thus, such testing involved laborious procedures and resulted in relatively high testing costs.

In recent years, provision of at-home testing services involving the sampling of one's own blood began. With such services, a test subject purchases a commercially available test kit, samples his or her own blood using a tool included in the kit, and mails the blood sample to a test institution. Thus, the test subject can later receive test results. However, blood that is sampled in such a manner rapidly becomes denatured with the elapse of time. This fact necessitates adequate processing, such as separation of blood cells immediately after the blood sampling. Also, the amount of the blood a subject can sample by himself or herself is limited. Thus, a means for efficiently recovering samples from sampled blood has been awaited. In order to satisfy such need, tools or apparatuses that can provide highly accurate test results via simpler operations have been developed.

JP Patent Publication (Kokai) No. 2001-321364 A discloses a tool for sampling and separating blood for self-blood sampling and a method for sampling one's own blood using such tool. With this method, blood is sampled and serum that is eluted from the sampled blood via spontaneous coagulation is then sampled. Since this method involves the use of a container with a large inner diameter, a large amount of sampled material is wasted. Accordingly, a large amount of blood is required (i.e., 200 to 300 µl) in order to obtain the amount of serum required for testing.

JP Patent Publication (Kokai) No. 2003-270239 A discloses a tool and a method for separating biological samples used for at-home testing. With this method, blood is sampled using a capillary, the sampled blood is applied dropwise to a cylinder containing a given amount of a diluent, and the blood is diluted, followed by separation of plasma from blood cells through a filter. With this method, however, it is difficult to obtain a given amount of sampled material, and errors are more likely to occur compared with conventional techniques. Since the test reagent is targeted to detect undiluted serum, test accuracy is lower in the case of diluted specimens, and the number of the test items and the test method are disadvantageously limited.

There is also an available at-home testing technique whereby a test subject samples his or her own blood, separates the sampled blood using a simple centrifuge included in the kit, and then mails the blood sample to a test institution (http://www.healthwave.co.jp/contsdc/gather.html). With this technique, however, the blood sample must be allowed to stand at room temperature for a given period of time (for approximately 30 minutes) in order to separate serum. Also, blood that adheres to the upper part of the tube or funnel is likely to coagulate, and the component that is eluted at the time of centrifugation is likely to contaminate the serum.

According to another at-home testing technique, a plasma separation filter membrane is impregnated with a trace amount of sampled blood, and the filter membrane is then mailed to a test institution (http://www.so-net.ne.jp/familyclinic/kenshin/aichi/menu aichi.html). With this technique, 3 or 4 drops of blood are applied dropwise to the plasma separation filter membrane and the blood is then tested. Because of the small amount of sampled blood, the number of the test items is limited (approximately 3 to 5 items). Also, extraction of the blood from the filter is laborious, and the amount of the sampled blood is unknown. Thus, test accuracy is low and the number of test items is limited.

### Disclosure of the Invention

The present invention has been made in order to overcome the drawbacks described above. Accordingly, it is an object of the present invention to provide a tool for recovering biological samples for realizing simpler and more accurate clinical testing with the use of trace amounts of blood samples and a method for recovering and separating biological samples using such tool.

The present inventors discovered that trace amounts of biological samples could be efficiently recovered and separated with the use of an absorption medium such as a nonwoven material or absorbent cotton.

Specifically, the present invention relates to a tool for recovering biological samples comprising: a recovery container body composed of an upper container portion having a medium-accommodating portion, provided with an upper opening, for accommodating and conserving an absorption medium to be impregnated with the recovered biological samples and a lower container portion for accommodating the biological samples separated from the medium; and a lid for closing the upper opening of the medium-accommodating portion, wherein the bottom of the medium-accommodating portion is provided with an open area in communication with the lower container portion.

Preferably, the tool for recovering biological samples is provided with an opening on the peripheral wall of the upper container portion to allow the content of the container body to communicate with the outer air for the following reason. That is, the provision of this opening accelerates impregnation of the absorption medium with the recovered biological samples.

According to an embodiment of the present invention, the upper container portion comprises: a cylindrical outer wall connected to the lower container portion; and a cylindrical inner wall fitted so as to be surrounded by the outer wall, comprising a flange portion at the upper end thereof, wherein the inner wall and the flange portion are rotatable with respect to and/or detachable from the outer wall, and the lid is fitted so as to cover the flange portion.

The outer wall and the inner wall are each provided with an opening at a given site, and the opening of the outer wall is aligned with that of the inner wall to form an opening to allow the content of the container body to communicate with the outer air at the upper container portion.

The inner diameter of the lower container portion is preferably smaller than that of the upper container portion. Particularly preferably, the inner diameter of the lower container portion is approximately 3 mm to 6 mm.

The present invention provides a tool for recovering biological samples, which further comprises an absorption medium to be impregnated with the recovered biological samples accommodated in the medium-accommodating portion.

As an absorption medium, woven or nonwoven material of polyester fibers and polyolefin fibers and/or melamine foam resin are particularly preferable.

Preferably, an absorption medium comprises an anticoagulant for inhibiting coagulation of recovered blood or the like.

Further, the lower portion of the recovery container is preferably filled with a separating medium in advance for assisting the separation of biological samples.

The present invention also provides a test kit comprising the tool for recovering biological samples according to the present invention and a lancet. This test kit may further comprise a centrifuge according to need.

Further, the present invention provides a method for recovering plasma with the use of the tool for recovering biological samples according to the present invention, wherein the absorption medium is impregnated with recovered blood, the opening of the container is closed, and the blood sample in the container is then subjected to centrifugation to transfer the blood from the absorption medium to the lower container portion, followed by separation and recovery of plasma.

With the use of the present invention, the step of recovering biological samples can be simplified, and the cost incurred for testing can be reduced. Since biological samples (e.g., plasma and blood cells) can remain completely separated from each other, deterioration of specimens over time can be inhibited, and testing can be carried out with high accuracy. Further, specimens can be efficiently obtained from small amounts of recovered blood, and thus, the burden imposed on a test subject at the time of blood sampling can be reduced.

This description includes part or all of the contents as disclosed in the description and/or drawings of Japanese Patent Application No. 2004-158290, which is a priority document of the present application.

### Brief Description of the Drawings

Fig. 1 schematically shows an overview of a tool for recovering biological samples according to an embodiment of the present invention.
Fig. 2 schematically shows a lid and a recovery container body of a tool for recovering biological samples according to an embodiment of the present invention, wherein the left figure represents a tool for recovering biological samples without an opening and the right figure represents a tool for recovering biological samples provided with an opening.
Fig. 3 shows constitutive members of a tool for recovering biological samples according to an embodiment of the present invention.
Fig. 4 shows structures of a tool for recovering biological samples according to an embodiment of the present invention.

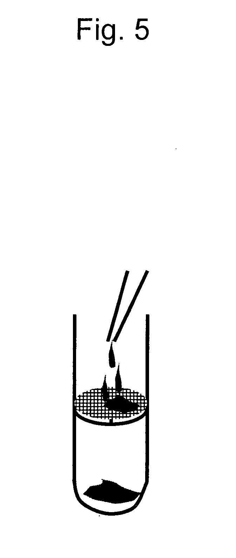

Fig. 5 schematically shows the experimentation of Example 1.
Fig. 6 is a chart showing the results of a comparison of the influences of an anticoagulant on the amount of residual medium.
Fig. 7 schematically shows the experimentation of Example 4.
Fig. 8 schematically shows how to use the tool for recovering biological samples according to the present invention.
Fig. 9 schematically shows the experimentation of Example 5.
Fig. 10A is a chart showing the results of a biochemical test (ALT) of plasma recovered with the use of the tool for recovering biological samples according to the present invention in comparison with serum recovered via a conventional technique.
Fig. 10B is a chart showing the results of a biochemical test (γ-GTP) of plasma recovered with the use of the tool for recovering biological samples according to the present invention in comparison with serum recovered via a conventional technique.
Fig. 10C is a chart showing the results of a biochemical test (T-CHO) of plasma recovered with the use of the tool for recovering biological samples according to the present invention in comparison with serum recovered via a conventional technique.
Fig. 10D is a chart showing the results of a biochemical test (TG) of plasma recovered with the use of the tool for recovering biological samples according to the present invention in comparison with serum recovered via a conventional technique.
Fig. 10E is a chart showing the results of a biochemical test (UA) of plasma recovered with the use of the tool for recovering biological samples according to the present invention in comparison with serum recovered via a conventional technique.
Fig. 10F is a chart showing the results of a biochemical test (CRE) of plasma recovered with the use of the tool for recovering biological samples according to the present invention in comparison with serum recovered via a conventional technique.

### Description of Reference Numerals

1: Tool for recovering biological samples
2: Recovery container body
3: Upper container portion
4: Lower container portion
5: Lid
6: Absorption medium
7, 7', and 7": Openings
8: Inner wall + flange portion
9: Medium-accommodating portion
10: Outer wall (peripheral wall)
11: Inner wall (peripheral wall)
12: Flange portion
13: Bottom of medium-accommodating portion
14: Inner diameter of lower container portion

### Preferred Embodiments of the Invention

Hereafter, the present invention is described in detail with reference to the drawings.

### 1. Tool for recovering biological samples

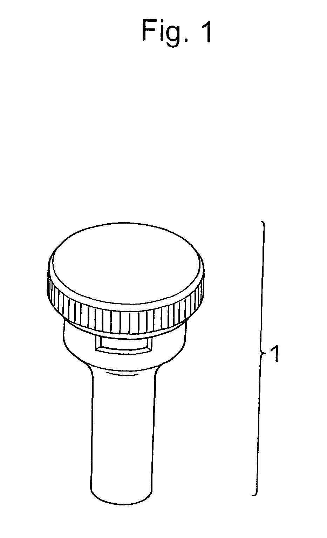

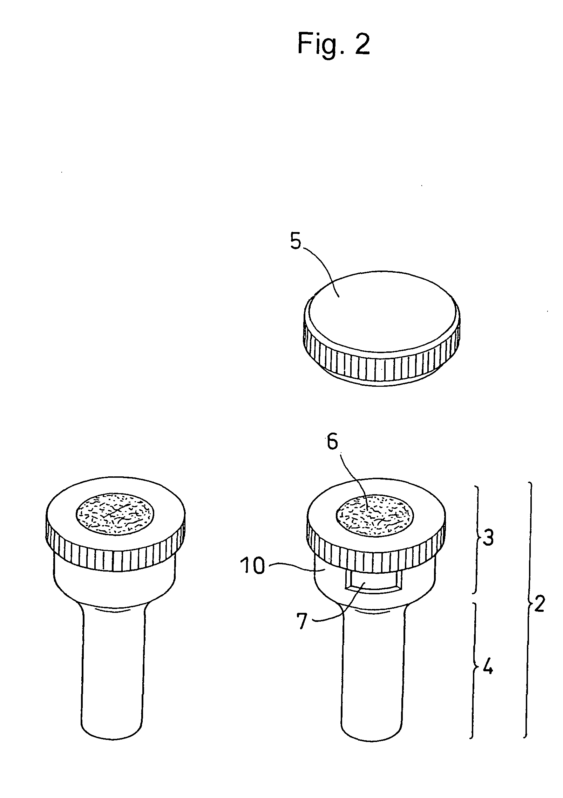

Fig. 1 to Fig. 4 each schematically show a tool for recovering biological samples according to an embodiment of the present invention. As shown in Fig. 2, the tool for recovering biological samples according to the present invention comprises: a container body 2 composed of an upper container portion 3 and a lower container portion 4; and a lid 5.

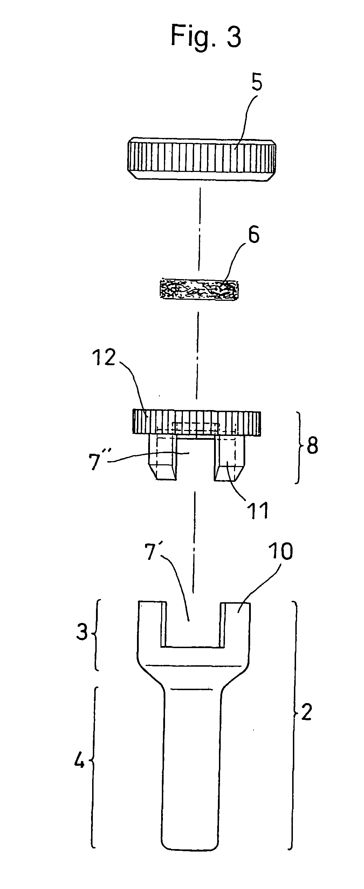

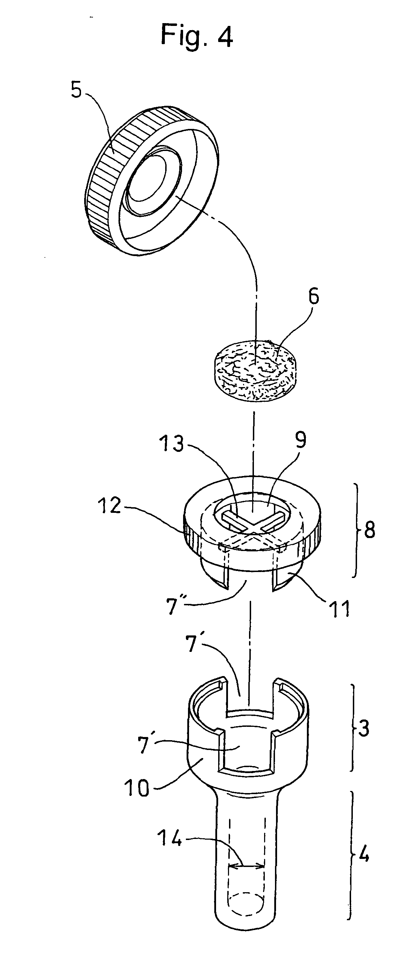

As shown in Fig. 4, the upper container portion 3 is provided with a medium-accommodating portion 9, provided with an upper opening, for accommodating and conserving an absorption medium 6 to be impregnated with recovered biological samples. The bottom of the medium-accommodating portion 9 is provided with an open area in communication with the lower container portion 4. Through such open area, biological samples that have been accommodated in the absorption medium 6 can be transferred to and accommodated in the lower container portion 4. Thus, an open area at the bottom of the medium-accommodating portion 9 may have any structure, as long as the absorption medium 6 is adequately accommodated therein and biological samples can be adequately transferred from the absorption medium to the lower container portion 4. For example, an open area at the bottom of the medium-accommodating portion 9 shown in Fig. 3 is provided with a cruciform structure 13; however, such structure may be of mesh or a radial form, as long as the aforementioned conditions are fulfilled.

The lid 5 is provided in order to close the upper opening of the medium-accommodating portion 9 and is fitted so as to cover the flange portion 12 at the upper container portion.

The peripheral wall 10 (and 11) of the upper container portion 3 is preferably provided with at least one opening 7 (7' and 7") that allows the content of the container body to communicate with the outer air. Provision of this opening 7 (7' and 7") accelerates impregnation of the absorption medium with the recovered biological samples. The number of openings may be one or more. In the example shown in Fig. 4, two openings are provided opposite each other. The size and the configuration of an opening are not particularly limited and may be adequately determined in accordance with the height of the upper container portion and operability, provided that the aforementioned conditions are satisfied.

As shown in Fig. 3 and in Fig. 4, the upper container portion 3 comprises: a cylindrical outer wall 10 connected to the lower container portion 4; and a cylindrical inner wall 11 fitted so as to be hermetically surrounded by the outer wall, comprising a flange portion 12 at the upper end thereof. The inner wall 11 and the flange portion 12 together form a member 8, which is rotatable with respect to and/or detachable from the outer wall 10, and the lid 5 is fitted so as to cover the flange portion 12.

The outer wall 10 and the inner wall 11 are provided with openings 7' and 7" at given portions, respectively, and the inner wall 11 is rotated with respect to the outer wall 10 to align the opening 7' with the opening 7". Thus, the opening 7 is provided at the upper container portion that allows the content of the container body 2 to communicate with the outer air. If the inner wall and the outer wall are turned so as to prevent the opening 7' from being aligned with the opening 7", the opening is closed and the communication between the inside and the outside of the container body 2 is blocked. As described below, the openings are kept open when the absorption medium is to be impregnated with biological samples. When centrifugation or other procedure is carried out thereafter, the openings are closed. The size of the opening 7' is not necessarily identical to that of the opening 7", as long as the aforementioned conditions are satisfied. However, they are preferably of substantially the same size and configuration.

Alternatively, the outer wall 10 may not be rotatable with respect to the inner wall 11. The openings may be blocked by increasing the height of the side surface of the lid 5, so that the lid 5 is fitted with the container.

The inner diameter 14 of the lower container portion 4 is preferably smaller than that of the upper container portion 3. The upper container portion 3 is required to have a size large enough to accommodate the absorption medium 6 and to allow the medium to absorb the biological samples upon dropwise application of the samples thereon (preferably 7 mm to 15 mm). This is because a smaller inner diameter is required in order to subject the sample to testing, while minimizing the waste of biological samples contained in the lower container portion 4. For example, the inner diameter of the lower container portion 4 is preferably approximately 3 mm to 6 mm.

As described above, the tool for recovering biological samples of the present invention is put to practical use while accommodating the absorption medium 6 in the medium-accommodating portion 9. The material of the absorption medium is required to be an inactive and safe absorptive material that would not react with biological samples, to rapidly absorb biological samples (e.g., blood), and to efficiently transfer the absorbed biological samples to the lower container portion 4 via centrifugation or the like.

The present inventors measured the time required for absorbing blood and the quantity of residual blood after centrifugation of various absorptive materials currently used in the medical field. Thus, they evaluated the adequacy of each of such materials as an absorption medium according to the present invention. As a result, they deduced that a woven or nonwoven material comprising rayon fibers, a woven or nonwoven material comprising polyester fibers and polyolefin fibers, and melamine foam resin would be adequate, and a woven or nonwoven material comprising polyester fibers and polyolefin fibers or melamine foam resin would be more adequate.

Preferably, the absorption medium further comprises an anticoagulant in order to prevent the recovered blood from being wasted due to coagulation and adsorption. Examples of an anticoagulant that can be used include, but are not limited to, EDTA-2Na, EDTA-1K, heparin-Na, Heparin-Li, sodium fluoride, sodium citrate, and an acid citrate dextrose (ACD) solution. The amount of the anticoagulant used is adequately determined in accordance with the relevant method described in the Examples below and in accordance with the amount of a recovered biological sample and the type of anticoagulant used. For example, desirable anticoagulation effects can be attained with the use of approximately 10 mg/ml of EDTA-2Na or approximately 0.1 mg/ml of heparin-Na. Materials that constitute a medium may be impregnated with such anticoagulant via soaking, adsorption, binding, or other adequate means.

The lower container portion 4 is preferably filled with a separating medium in advance for assisting the separation of biological samples (e.g., blood). Such separating medium is known in the art, and a commercialized product can be easily obtained. Examples of a separating medium include, but are not limited to, sodium diatrizoate, polysaccharide, dextran, polyester gel, acrylic resin, and a mixture thereof. The amount of a separating medium introduced into the container is determined in accordance with the amount of a recovered biological sample and the type of separating medium.

When an individual samples his or her one's own blood by needling, the amount of blood that can be sampled is approximately 2 to 5 drops (approximately 60 to 150 µl), although such amount varies between individuals. If 50 µl of plasma (approximately 100 µl in terms of whole blood) is available, 16 items of a general biochemical test can be carried out. When blood is sampled with the use of the tool for recovering biological samples according to the present invention, plasma can be efficiently separated from a trace amount of blood (approximately 100 µl), and such plasma can be subjected to testing without any waste thereof.

### 2. Test kit

The present invention provides a test kit comprising the tool for recovering biological samples according to the present invention, a lancet, and a centrifuge.

The "lancet" is a needle used for blood sampling by needling. Various types of lancets for recovering trace amounts of blood through a finger, earlobe, sole, or the like are manufactured and sold. A person skilled in the art can adequately make use of such a lancet for the kit of the present invention.

The centrifuge is not particularly limited, provided that it can transfer blood that has impregnated the absorption medium 6 of the tool for recovering biological samples according to the present invention to the lower container portion 4 and that it can separate blood cells from the blood. In particular, such centrifuge is preferably of a small size and a simple structure, so that it is readily used at locations other than medical institutions (e.g., in the home of a test subject). Such centrifuges are already used for some forms of at-home testing or the like. A person skilled in the art can adequately modify such a centrifuge to result in a centrifuge that is suitable as a tool for recovering biological samples according to the present invention.

The kit of the present invention may comprise other adequate components, in addition to the aforementioned components, according to need. Examples of such components include cottons impregnated with ethyl alcohol for disinfection and first-aid adhesive tape for arresting hemorrhage.

The tool for recovering biological samples according to the present invention is of a compact size, and it can efficiently recover and separate a trace amount of blood. Accordingly, the test kit of the present invention is suitably used for at-home test services involving the sampling of an individual's own blood.

### 3. Method for recovering biological samples

The present invention provides a method for recovering biological samples with the use of the tool for recovering biological samples of the present invention. In this method, the absorption medium 6 is impregnated with the recovered blood, the opening of the container is closed, and the blood sample in the container is subjected to centrifugation to transfer the blood from the absorption medium 6 to the lower container portion 4, followed by separation and recovery of plasma.

Blood sampling is carried out using an adequate lancet via a finger, earlobe, or sole, for example. In order to minimize the amount of the recovered blood being wasted, blood is preferably applied dropwise directly from the site of sampling to the absorption medium 6. In such a case, the opening 7 is kept open in order to accelerate the absorption of the blood by the medium. As described above, the absorption medium can be impregnated with an anticoagulant in advance. Thus, the blood absorbed by the medium can be subjected to the following step of separation without undergoing coagulation or denaturation.

When the blood has been completely absorbed by the absorption medium, the lid 5 is fitted outer the upper container portion to completely close the opening, and the blood sample in the container is then subjected to centrifugation. Via centrifugation, the blood absorbed by the absorption medium 6 is transferred to the lower container portion 4, and simultaneously, blood is divided into blood cells and plasma. By filling the lower container portion with a separating medium in advance, recontamination of the separated plasma by blood cells can be prevented, as described above. The biological samples that have been divided into blood cells and plasma can be subjected to various clinical tests in suitable conditions without denaturation.

### Examples

### Example 1: Selection of blood-absorbing material (medium)

### 1. Method

In order to select an optimal blood-absorbing material, 10 types of media (Table 1) used for various applications were inspected in terms of absorptivity and the quantity of residual blood. The media were each cut into pieces with diameters of 12 mm each, and the pieces were mounted in experimental tubes. Blood recovered with the aid of heparin was applied dropwise in amounts of 400 µl each, the time required for absorption was inspected, and the conditions of the blood remaining after the centrifugation (at room temperature and 2,500 rpm for 5 minutes) were visually inspected. Blood samples recovered from 2 healthy volunteers, i.e., blood sample A and blood sample B, were used in this procedure.

**Table 1**

| Materials inspected | | |
|---|---|---|
| | Medium | Composition* |
| (i) | B | Polyester |
| (ii) | D | Rayon |
| (iii) | E | Acrylic/polyolefin |
| (iv) | F | Rayon/polyolefin |
| (v) | G | Polyolefin |
| (vi) | H | Polyester/polyolefin/rayon |
| (vii) | I | Polyester/nylon |
| (viii) | J | Rayon/acrylic binder |
| (ix) | K | Polyester/polyolefin |
| (x) | L | Melamine |

### 2. Results (results of observing the absorption conditions and the quantity of residual blood after centrifugation)

Among the 10 types of materials inspected, 4 types of materials did not absorb blood, 3 types of materials slowly absorbed blood, and 3 other types satisfactorily absorbed blood, as shown in Table 2. In a material that was less likely to absorb blood, the quantity of residual blood after centrifugation was likely to be small. In 2 of the 3 materials that satisfactorily absorbed blood, the quantity of residual blood was adequate (media K and L).

**Table 2**

| | Medium | Blood sample A | | Blood sample B | |
|---|---|---|---|---|---|
| | | Absorption time | Residual quantity after centrifugation | Absorption time | Residual quantity after centrifugation |
| (i) | B | × | 2 | × | 3 |
| (ii) | D | 120 sec | 1 | 60 sec | 2 |
| (iii) | E | × | 3 | × | 3 |
| (iv) | F | 120 sec | 3 | 120 sec | 1 |
| (v) | G | × | 5 | × | 5 |
| (vi) | H | × | 3 | × | 3 |
| (vii) | I | 120 sec | 1 | 120 sec | 1 |
| (viii) | J | 50 sec | 1 | 80 sec | 1 |
| (ix) | K | 20 sec | 2 | 25 sec | 4 |
| (x) | L | 60 sec | 3 | 15 sec | 3 |

| | | | | | |
|---|---|---|---|---|---|
| * An absorption time represented by "x" indicates that blood was not absorbed after 120 sec. * The quantity of residual blood after centrifugation was evaluated at five levels. Larger numbers indicate larger residual quantities. | | | | | |

### Example 2: Influences of anticoagulant

The two types of media selected in Example 1 (i.e., media K and L) were impregnated with a given amount of an anticoagulant, such as heparin-Na, to inspect changes in absorption quantity and residual quantity.

### 1. Method

Media K and L were each impregnated with a solution containing 0.1 mg/ml of heparin-Na (adjusted with purified water) (0.01 to 0.1 mg/ml in the case of a general blood sampling tube), dehydrated at room temperature overnight, and then cut into pieces each having a diameter of 12 mm. The blood samples (400 µl) recovered with the use of blood sampling tubes were applied dropwise to the media, allowed to stand at room temperature at 0 minute, 10 minutes, and 20 minutes after initiation, and then centrifuged at room temperature and 2,500 rpm for 5 minutes, in order to inspect the influences of haemolysis. The weights of the components onto which the media had been mounted were measured in advance, and the weights were also measured after centrifugation to compare the quantities of absorption. The blood samples were ice-cooled immediately after sampling, transported, and then subjected to the experiment. Thus, the experiment was initiated approximately 15 to 20 minutes after blood sampling.

### 2. Results

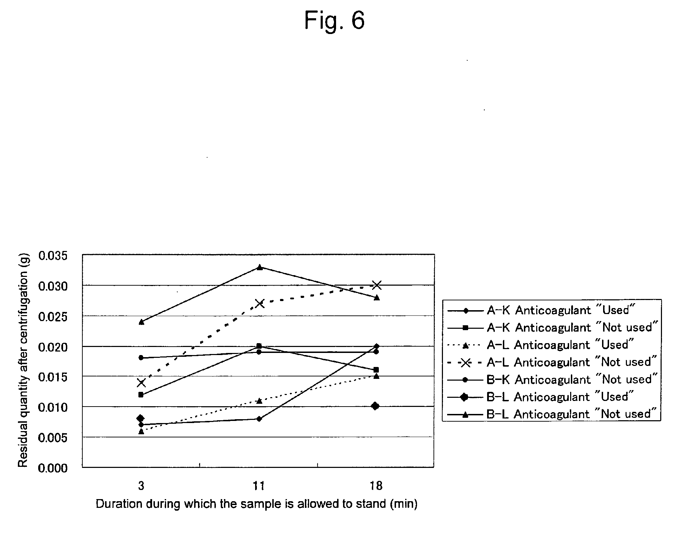

As shown in Table 3 and Fig. 6, the residual quantity after centrifugation due to coagulation was smaller when the medium impregnated with heparin-Na was used, compared with the cases in which the medium was not impregnated with heparin-Na was used. Compared with medium K, the influences of an anticoagulant on medium L were smaller. That is, coagulated blood was more likely to remain after centrifugation.

**Table 3**

| Influences of anticoagulant | | | | | | |
|---|---|---|---|---|---|---|
| Blood sample | Medium | Anticoagulant | Time before centrifugation | Weight before centrifugation (g) | Weight after centrifugation (g) | Differences (g) |
| Blood sample A | K | Used | 3 min | 1.651 | 1.658 | 0.007 |
| | K | Not used | 3 min | 1.655 | 1.667 | 0.012 |
| | L | Used | 3 min | 1.649 | 1.655 | 0.006 |
| | L | Not used | 3 min | 1.656 | 1.670 | 0.014 |
| | K | Used | 11 min | 1.651 | 1.659 | 0.008 |
| | K | Not used | 11 min | 1.653 | 1.673 | 0.020 |
| | L | Used | 11 min | 1.650 | 1.661 | 0.011 |
| | L | Not used | 11 min | 1.647 | 1.674 | 0.027 |
| | K | Used | 18 min | 1.654 | 1.674 | 0.020 |
| | K | Not used | 18 min | 1.657 | 1.673 | 0.016 |
| | L | Used | 18 min | 1.653 | 1.668 | 0.015 |
| | L | Not used | 18 min | 1.656 | 1.686 | 0.030 |
| Blood sample B | K | Used | Not inspected | | | |
| | K | Not used | 3 min | 1.657 | 1.675 | 0.018 |
| | L | Used | 3 min | 1.648 | 1.656 | 0.008 |
| | L | Not used | 3 min | 1.651 | 1.675 | 0.024 |
| | K | Used | Not inspected | | | |
| | K | Not used | 11 min | 1.658 | 1.677 | 0.019 |
| | L | Used | Not inspected | | | |
| | L | Not used | 11 min | 1.655 | 1.688 | 0.033 |
| | K | Used | Not inspected | | | |
| | K | Not used | 18 min | 1.660 | 1.679 | 0.019 |
| | L | Used | 18 min | 1.656 | 1.666 | 0.010 |
| | L | Not used | 18 min | 1.647 | 1.675 | 0.028 |

### Example 3: Type and concentration of anticoagulant and time before centrifugation

### 1. Method

In addition to the materials K and L selected in Example 2 (a nonwoven material of polyester and polyolefin and melamine foam resin, respectively), material K' (a material composed of the same material as medium K but with a texture coarser than that of medium K) was immersed in solutions each containing 0.1 mg/ml and 1.0 mg/ml of heparin-Na (adjusted with purified water) and solutions each containing 1.0 mg/ml and 10.0 mg/ml of EDTA-2Na (adjusted with purified water), dehydrated at room temperature overnight, cut into pieces each with diameters of 12 mm, and mounted in experimental tubes (2 pieces in each tube). The blood (800 µl) sampled with the use of a plain blood sampling tube was applied dropwise thereto, not allowed to stand or allowed to stand at room temperature for 30 minutes, and then centrifuged at room temperature and 2,500 rpm for 5 minutes. The weights of the components onto which the media had been mounted were measured in advance, and the weights were also measured after centrifugation to compare the quantities of absorption.

### 2. Results

**Table 4**

| Medium | Anticoagulant | Conc. (mg/ml) | Time before centrifugation | Previous weight (g) | Post-centrifugation weight (g) | Difference (g) | Time difference (g) |
|---|---|---|---|---|---|---|---|
| L | None | | 0 | 1.6451 | 1.6482 | 0.0031 | |
| | | | 30 | 1.6491 | 1.6714 | 0.0223 | 0.0192 |
| | heparin-Na | 0.1 | 0 | 1.6483 | 1.6515 | 0.0032 | |
| | | | 30 | 1.6477 | 1.6610 | 0.0133 | 0.0101 |
| | | 1.0 | 0 | 1.6588 | 1.6632 | 0.0044 | |
| | | | 30 | 1.6561 | 1.6637 | 0.0076 | 0.0032 |
| | EDTA-2Na | 1.0 | 0 | 1.6565 | 1.6593 | 0.0028 | |
| | | | 30 | 1.6491 | 1.6678 | 0.0187 | 0.0159 |
| | | 10.0 | 0 | 1.6544 | 1.6537 | -0.0007 | |
| | | | 30 | 1.6621 | 1.6720 | 0.0099 | 0.0106 |
| K | None | | 0 | 1.6590 | 1.6616 | 0.0026 | |
| | | | 30 | 1.6666 | 1.6874 | 0.0208 | 0.0182 |
| | heparin-Na | 0.1 | 0 | 1.6615 | 1.6649 | 0.0034 | |
| | | | 30 | 1.6626 | 1.6662 | 0.0036 | 0.0002 |
| | | 1.0 | 0 | 1.6743 | 1.6758 | 0.0015 | |
| | | | 30 | 1.6624 | 1.6642 | 0.0018 | 0.0003 |
| | EDTA-2Na | 1.0 | 0 | 1.6658 | 1.6670 | 0.0012 | |
| | | | 30 | 1.6736 | 1.6819 | 0.0083 | 0.0071 |
| | | 10.0 | 0 | 1.6690 | 1.6680 | -0.0010 | |
| | | | 30 | 1.6677 | 1.6706 | 0.0029 | 0.0039 |
| K' | None | | 0 | 1.6557 | 1.6571 | 0.0014 | |
| | | | 30 | 1.6565 | 1.6658 | 0.0093 | 0.0079 |
| | heparin-Na | 0.1 | 0 | 1.6550 | 1.6582 | 0.0032 | |
| | | | 30 | 1.6567 | 1.6585 | 0.0018 | -0.0014 |
| | | 1.0 | 0 | 1.6607 | 1.6628 | 0.0021 | |
| | | | 30 | 1.6591 | 1.6615 | 0.0024 | 0.0003 |
| | EDTA-2Na | 1.0 | 0 | 1.6595 | 1.6614 | 0.0019 | |
| | | | 30 | 1.6643 | 1.6666 | 0.0023 | 0.0004 |
| | | 10.0 | 0 | 1.6675 | 1.6674 | -0.0001 | |
| | | | 30 | 1.6587 | 1.6591 | 0.0004 | 0.0005 |

As shown in Table 4, the residual quantities after centrifugation were as follows: medium K' < medium K < medium L. In the absence of an anticoagulant, as well, the residual quantities 30 minutes later were as follows: medium K' < medium K < medium L, although the difference between medium K and medium L was insignificant. The quantity of residual medium K' was about a half that of medium K or L.

The influences of an anticoagulant on the reduction of the quantity of absorption by medium K and by medium K' were observed with the addition of 0.1 or 1.0 mg/ml of heparin-Na or 10.0 mg/ml of EDTA-2Na; i.e., the difference between conditions at 0 minute and at 30 minute was small. However, the influence was insignificant with the addition of 1.0 mg/ml of EDTA-2Na. In contrast, the influences of an anticoagulant on medium L were insignificant at any concentration.

In serum and plasma after centrifugation, pudding-like coagulation was observed without the addition of an anticoagulant and with the addition of 1.0 mg/ml of EDTA-2Na. However, no coagulation was observed with the addition of heparin-Na or 10.0 mg/ml of EDTA-2Na.

### Example 4: Configuration of container (opening)

### 1. Method

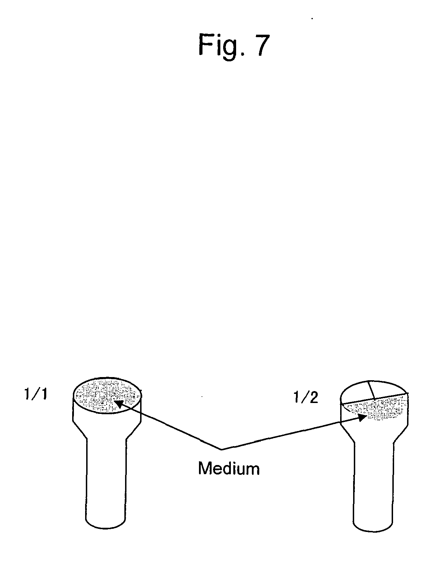

The present inventors believed that the absence of air discharge was a major cause of unsatisfactory absorption in a given medium. Thus, the size of the medium was reduced to 1/2 and to 1/4, ventilation openings were provided at the upper portion thereof, and blood absorption was compared, as shown in Fig. 7. Medium K containing 1 mg/ml of heparin-Na was used, and a 1/1-sized membrane (without an opening) was used as a control to carry out a similar experiment (Fig. 7).

### 2. Results

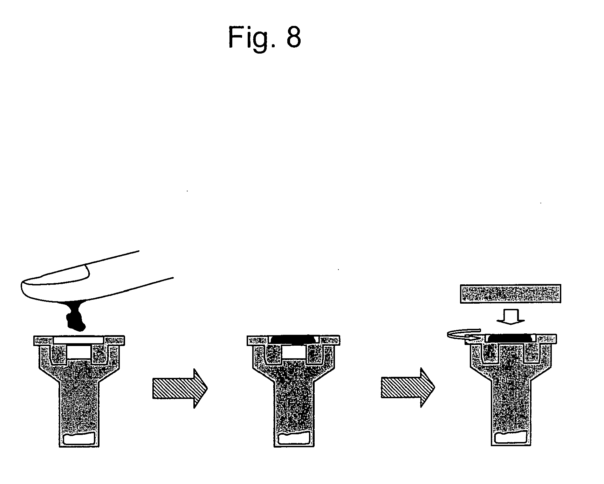

Both the 1/2-sized and 1/4-sized membranes exhibited satisfactory blood absorption. If an opening was provided at the upper portion of the medium, blood that had adhered to the opening would be disadvantageously dried. Thus, an opening was provided on the side surface of the container (Fig. 8).

### Example 5: Configuration of container (inner diameter)

The amount of whole blood sampled in the blood-sampling container is approximately 100 µl (approximately 50 µl in terms of serum or plasma). In general, approximately 50 µl of serum or plasma is required in order to carry out 16 items of a general biochemical test. Thus, the optimal inner diameter of the container for extracting 50 µl of serum or plasma from a supernatant after centrifugation while minimizing the waste of samples was determined.

### 1. Preliminary examination

Calculation of liquid surface depending on the tube inner diameter, provided that the amount of the supernatant was 50 µl.
When the inner diameter is 3 mm: 50 ÷ (1.5X1.5X3.14) = 7.08 mm (height)
When the inner diameter is 4 mm: 50 ÷ (2.0X2.0X3.14) = 3.98 mm (height)
When the inner diameter is 5 mm: 50 ÷ (2.5X2.5X3.14) = 2.55 mm (height)

Thus, a tube inner diameter must be no greater than 4 mm in order to smoothly recover samples.

### 2. Method

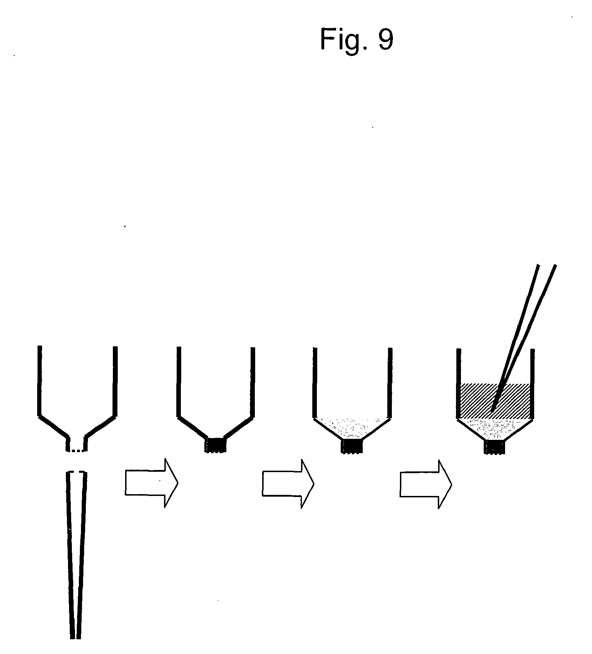

The apexes of fractionation chips (inner diameter: approximately 4 mm) were cut off, 40 µl to 60 µl of a separating medium (PS-gel 460, Nippon Paint Co., Ltd.) was introduced into each thereof, the openings of which had been closed with the use of solder, centrifugation was mildly carried out, and chips containing a separating medium with flattened top surfaces were prepared. The control serum samples were independently introduced thereinto in amounts of 40 µl, 50 µl, and 60 µl, and the quantity of samples that could be recovered was examined with the use of a micropipetting chip (capable of recovering up to approximately 200 µl) (Fig. 9).

### 3. Results

It was impossible to recover 35 µl of samples from 40 µl of samples. It was difficult to recover 40 µl of samples from 50 µl of samples. However, it was possible to recover 45 µl of samples from 60 µl of samples.

As a result, the dead volume was found to be approximately 15 µl when recovering samples from a fractionation chip having an inner diameter of approximately 4 mm. Thus, it was deduced that a plasma component sampled from trace amounts of self-sampled blood could be efficiently subjected to clinical testing (i.e., 100 µl of whole blood (approximately 50 µl in terms of serum and plasma)). When the samples were mounted on an autoanalyzer (i.e., such that a sample probe automatically undergoes sampling), a certain liquid level would be required. Accordingly, a small inner diameter of approximately 4 mm was considered to be suitable.

### Example 6: Comparison of the results of biochemical testing of plasma recovered with the use of the tool for recovering biological samples of the present invention and serum obtained via conventional blood sampling

### 1. Method

The plasma recovered with the use of the tool for recovering biological samples of the present invention (i.e., the plasma that was absorbed by medium K containing 1 mg/ml of heparin-Na, centrifuged, and then recovered) and the serum obtained via conventional blood sampling (i.e., the serum that was recovered by centrifugation in an experimental tube without being absorbed by a medium) were subjected to 16 items of a general biochemical test. The results were compared.

The test items were as follows.
1: AST; 2: ALT; 3: γ-GTP; 4: T-CHO; 5: TG; 6: HDL-C; 7: UA; 8: GLU; 9: CRE; 10: UN; 11: ALP; 12: TP; 13: ALB; 14: T-BIL; 15: LDH; 16: AMY

### 2. Results

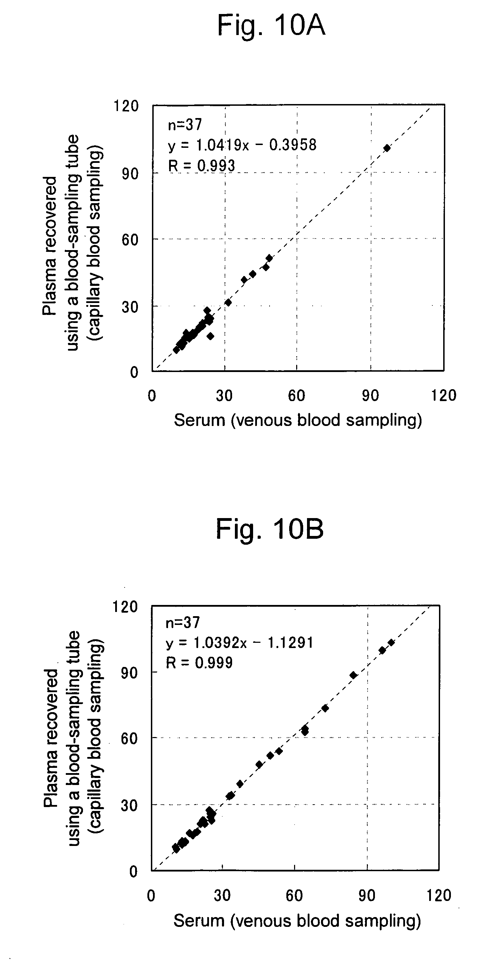

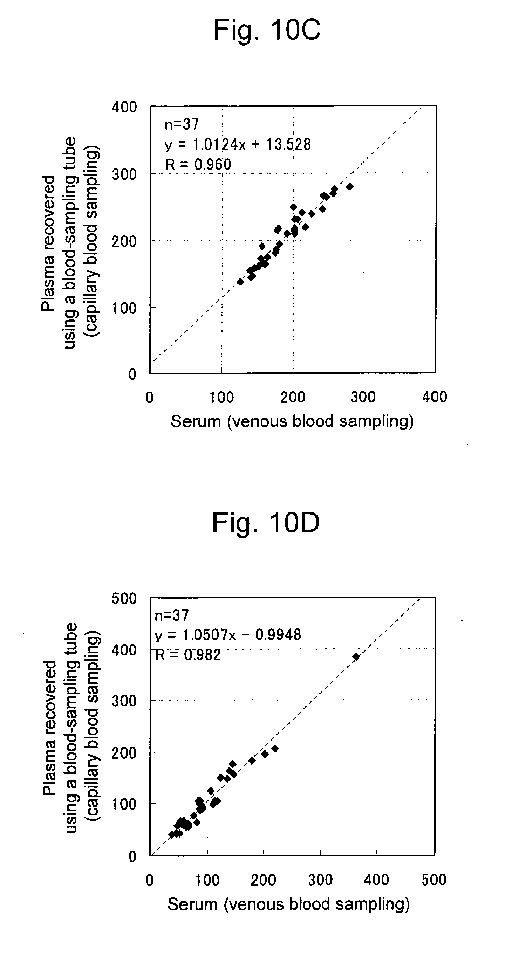

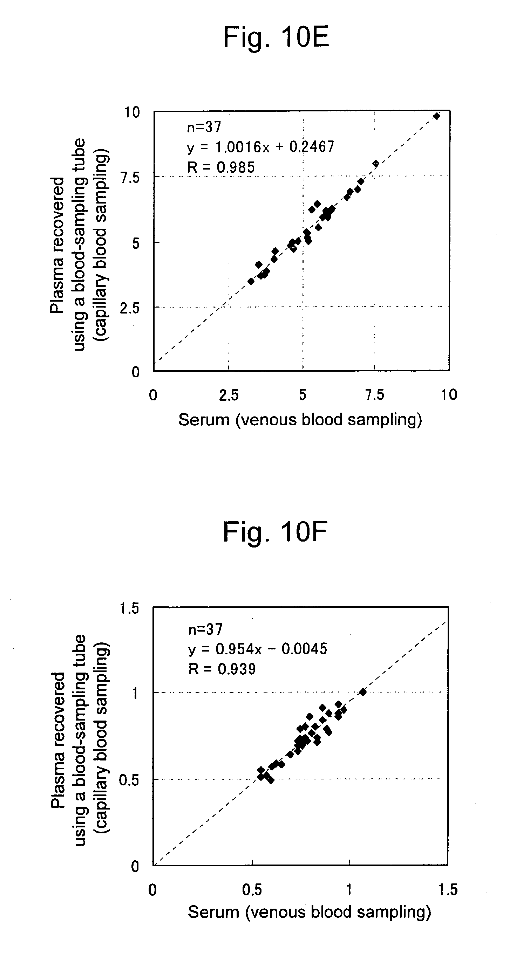

As shown in Fig. 10, the correlation of the measured values between the plasma recovered with the use of the tool for recovering biological samples of the present invention and the serum obtained via conventional blood sampling was found to be very strong.

### Industrial Applicability

The tool for recovering biological samples according to the present invention enables efficient recovery of specimens from small amounts of sampled blood. This can reduce the burden imposed on a test subject at the time of blood sampling. Since biological samples (e.g., plasma and blood cells) can be completely separated via simple operations, denaturation of specimens with the elapse of time can be inhibited, and testing can be carried out with high accuracy. The tool for recovering biological samples according to the present invention is of a compact size. Thus, such tool is suitably used for at-home testing or the like, and it can also be suitably used for blood sampling from babies or children, where blood sampling is usually difficult.

All publications, patents, and patent applications cited herein are incorporated herein by reference in their entirety.

## Claims

1. A tool for recovering biological samples comprising: a recovery container body composed of an upper container portion having a medium-accommodating portion, provided with an upper opening, for accommodating and conserving an absorption medium to be impregnated with the recovered biological samples and a lower container portion for accommodating the biological samples separated from the medium; and a lid for closing the upper opening of the medium-accommodating portion, the bottom of such medium-accommodating portion being provided with an open area in communication with the lower container portion.

2. The tool for recovering biological samples according to claim 1, wherein an opening is provided on the peripheral wall of the upper container portion to allow the content of the container body to communicate with the outer air.

3. The tool for recovering biological samples according to claim 1 or 2, wherein the upper container portion comprises: a cylindrical outer wall connected to the lower container portion; and a cylindrical inner wall fitted so as to be surrounded by the outer wall, comprising a flange portion at the upper end thereof, wherein the inner wall and the flange portion are rotatable with respect to and/or detachable from the outer wall, and the lid is fitted so as to cover the flange portion.

4. The tool for recovering biological samples according to claim 3, wherein the outer wall and the inner wall are each provided with an opening at a given site, and the opening of the outer wall is aligned with that of the inner wall to form an opening to allow the content of the container body to communicate with the outer air at the upper container portion.

5. The tool for recovering biological samples according to any one of claims 1 to 4, wherein the inner diameter of the lower container portion is smaller than that of the upper container portion.

6. The tool for recovering biological samples according to claim 5, wherein the inner diameter of the lower container portion is 3 mm to 6 mm.

7. The tool for recovering biological samples according to any one of claims 1 to 6, which comprises an absorption medium to be impregnated with the recovered biological samples accommodated in the medium-accommodating portion.

8. The tool for recovering biological samples according to claim 7, wherein the absorption medium is composed of a woven or nonwoven material of polyester fibers or polyolefin fibers, melamine foam resin, or a combination thereof.

9. The tool for recovering biological samples according to claim 7 or 8, wherein the absorption medium comprises an anticoagulant.

10. The tool for recovering biological samples according to any one of claims 1 to 9, wherein the lower portion of the recovery container is filled with a separating medium in advance.

11. A test kit comprising the tool for recovering biological samples according to any one of claims 7 to 10 and a lancet.

12. The test kit according to claim 11, which further comprises a centrifuge.

13. A method for recovering plasma with the use of the tool for recovering biological samples according to any one of claims 7 to 10, wherein the absorption medium is impregnated with the recovered blood, the opening of the container is closed, and the blood sample in the container is then subjected to centrifugation to transfer the blood from the absorption medium to the lower container portion, followed by separation and recovery of plasma.
